(19) Europäisches Patentamt
European Patent Office
Office européen des brevets

(11) Numéro de publication: **0 307 285**
**A1**

(12) **DEMANDE DE BREVET EUROPEEN**

(21) Numéro de dépôt: **88402198.1**

(22) Date de dépôt: **01.09.88**

(51) Int. Cl.$^4$: **C 12 N 5/00**
C 12 N 15/00, C 12 P 21/02

(30) Priorité: **01.09.87 FR 8712166**

(43) Date de publication de la demande:
**15.03.89 Bulletin 89/11**

(84) Etats contractants désignés:
**AT BE CH DE ES FR GB GR IT LI LU NL SE**

(71) Demandeur: **SANOFI, société anonyme**
**40, Avenue George V**
**F-75008 Paris (FR)**

(72) Inventeur: **Lupker, Johannes**
**9, rue Léon Viala**
**F-31520 Ramonville-Saint-Agne (FR)**

**Miloux, Brigitte**
**Chemin de Safrana**
**F-31450 Montgiscard (FR)**

**Roskam, Willem**
**Majouret**
**F-31450 Montgiscard (FR)**

(74) Mandataire: **Gillard, Marie-Louise et al**
**Cabinet Beau de Loménie 55, Rue d'Amsterdam**
**F-75008 Paris (FR)**

(54) **Cellules eucaryotes recombinées productrices, d'interleukine-2, procédé et vecteurs pour leur obtention et procédé d'obtention d'interleukine-2.**

(57) L'invention concerne des cellules eucaryotes recombinées productrices d'interleukine-2 qui contiennent, avec les moyens nécessaires à leur expression, une séquence d'ADN codant pour la dihydrofolate réductase et une séquence d'ADN codant pour un précurseur hybride de l'interleukine-2 dont le peptide-signal est celui de l'un des précurseurs naturels de l'hormone de croissance humaine.

L'invention concerne également le procédé et les vecteurs pour l'obtention de ces cellules ainsi que le procédé d'obtention d'interleukine-2 à partir de celles-ci.

Application : Production d'interleukine-2.

EP 0 307 285 A1

## Description

## Cellules eucaryotes recombinées productrices d'interleukine-2, procédé et vecteurs pour leur obtention et procédé d'obtention d'interleukine-2.

La présente invention concerne des cellules eucaryotes recombinées productrices d'interleukine-2. Elle concerne également un procédé et les vecteurs pour l'obtention de ces cellules. Enfin, elle a aussi pour objet l'obtention d'interleukine-2 par mise en culture desdites cellules.

Les cellules eucaryotes selon l'invention contiennent, avec les moyens nécessaires à leur expression, une séquence d'ADN codant pour la dihydrofolate réductase et une séquence d'ADN codant pour un précurseur hybride de l'interleukine-2 dont le peptide-signal est celui de l'un des précurseurs naturels de l'hormone de croissance humaine.

Ces cellules eucaryotes recombinées sont obtenues par le procédé qui consiste à transfecter des cellules eucaryotes à l'aide d'un vecteur portant, avec les moyens nécessaires à leur expression, une séquence d'ADN codant pour la dihydrofolate réductase et une séquence d'ADN codant pour un précurseur hybride de l'interleukine-2 dont le peptide-signal est celui de l'un des précurseurs naturels de l'hormone de croissance humaine et à sélectionner les cellules transfectées, productrices d'interleukine-2, par croissance dans des milieux successifs, présentant l'un par rapport au précédent une concentration accrue en méthotrexate.

L'interleukine-2 est une lymphokine : elle est sécrétée par les lymphocytes-T matures des mammifères en réponse à une activation par un antigène ou un composé mitogène. Elle joue un rôle essentiel en agissant, sur la prolifération et la différentiation de différents types de cellules impliquées dans les réponses immunitaires (Robb, R.J. (1984) Immunol. Today, 5, 203-209).

L'interleukine-2 d'origine humaine a été plus particulièrement étudiée. On sait qu'il s'agit d'une protéine de 133 acides aminés portant, fixé sur le résidu thréonine en position 3, un tétrasaccharide (Conradt, H.S. et al (1986) Carbohydr. Res., 149, 443-450). Les lymphocytes-T matures la synthétisent d'abord sous la forme d'un précurseur de 153 acides aminés et la sécrètent après élimination par coupure au niveau du réticulum endoplasmique, du peptide-signal de 20 acides aminés, puis après glycosylation dans l'appareil de Golgi, sous la forme d'une protéine de 133 acides aminés - dite protéine mature glycosylée.

Les propriétés biologiques de l'interleukine-2 d'origine humaine la destinent à une utilisation en tant que principe actif de médicaments utiles pour le traitement de maladies telles que les cancers et certaines maladies infectieuses ou parasitaires. Pour cette utilisation il paraît préférable de disposer d'interleukine-2 sous une forme glycosylée : la chaîne latérale saccharidique paraît contribuer en effet à stabiliser la protéine et à en améliorer la tolérance lors de son administration aux patients.

L'obtention d'interleukine-2 à partir soit de lymphocytes périphériques sains (Kniep, E.M. et al (1984) Eur. J., Biochem., 143, 199-203), soit d'une lignée cellulaire lymphoblastoïde telle que la lignée Jurkat (Robb, R.J. et al (1983) Proc. Natl. Acad. Sci. USA., 80, 5990-5994) a été décrite.

Les méthodes mises en oeuvre se sont avérées peu adaptées en raison tant de difficultés liées à la culture de lymphocytes sains que de la nécessité d'une phase d'induction.

Consécutivement au clonage d'un ADN complémentaire codant pour l'interleukine-2 (Taniguchi, T. et al (1983) Nature, 302, 305-310) l'utilisation de micro-organismes rendus aptes à la production d'interleukine-2 grâce aux techniques de l'ingénierie génétique a été décrite. La demande de brevet EP-A-0089 062 montre la possibilité d'utiliser - outre la bactérie Escherichia coli incapable de glycosylation - les cellules de singe COS. La demande de brevet EP-A-0172619 présente l'utilisation de diverses autres cellules animales parmi lesquelles notamment des cellules d'ovaire de hamster chinois (cellules CHO).

Il est encore connu que pour permettre, à l'issue d'une transfection, la mise en évidence, au sein d'une population de cellules eucaryotes, des cellules qui ont effectivement intégré un vecteur particulier, il est utile que ledit vecteur porte une séquence d'ADN dont l'expression puisse conférer aux cellules transfectées un avantage sélectif.

Une séquence d'ADN particulièrement adaptée est une séquence codant pour la dihydrofolate réductase (enzyme ci-après désignée par l'abréviation dhfr) ; décrite par Subramani, et al ((1981) Mol. Cel. Biol., 854-864) pareille séquence, portée par un vecteur d'expression, permet, après transfection de cellules incapables de synthétiser sous une forme fonctionnelle de la dhfr (cellules DHFR-), la croissance sur un milieu déficient en hypoxanthine, en glycine et en thymidine, des seules cellules ayant effectivement incorporé le vecteur.

L'intérêt accordé à l'utilisation d'un vecteur portant, avec les moyens nécessaires à son expression, une séquence d'ADN codant pour la dhfr est accentué par le fait qu'elle peut être à l'origine, que la cellule eucaryote transfectée soit capable (cellules DHFR+) ou non (cellules DHFR-) de synthétiser sous une forme fonctionnelle la dhfr, d'un processus d'amplification conduisant à une obtention accrue d'une protéine d'interêt codée par une séquence d'ADN portée, avec les moyens nécessaires à son expression, par ledit vecteur. Le mécanisme de cette amplification reste à préciser. On sait que la dhfr est inhibée par le composé connu sous l'appellation méthotrexate (acide N-((((diamino-2,4 ptéridinyl-6) méthyl) méthylamino)-4 benzoyl) L-glutamique) et que la présence de méthotrexate dans un milieu de culture sélectif entraîne la mort de la plupart des cellules et que seules subsistent des cellules devenues capables de synthétiser des quantités importantes de dhfr. Et il a été constaté (Kaufman, R.J. et al (1982) J. Mol. Biol., 159, 601-621), chez des cellules ayant incorporé un vecteur portant, avec les moyens necessaires à leur expression, une séquence d'ADN codant pour la dhfr et une séquence d'ADN codant pour une autre protéine, que cette production amplifiée de dhfr était accompagnée d'une production amplifiée de ladite protéine.

La demanderesse, ayant construit des vecteurs portant simultanément, avec les moyens nécessaires à leur expression, une séquence d'ADN codant pour un précurseur naturel d'une interleukine-2 et une séquence d'ADN codant pour la dhfr, a constaté lors de la culture de cellules eucaryotes (et notamment de cellules CHO) ayant incorporé l'un des vecteurs concernés, que de tels vecteurs ne permettaient, en conditions d'expression transitoire, c'est-à-dire avant sélection d'une lignée hautement productrice par culture dans des milieux successifs présentant l'un par rapport au précédent une concentration accrue en méthotrexate, le recueil à partir du milieu de culture que de quantités d'interleukine-2 inférieures à celles recueillies à partir du milieu de culture des cellules de même nature ayant incorporé un vecteur ne différant que par l'absence de la séquence d'ADN codant pour la dhfr et des moyens nécessaires à l'expression de cette séquence.

Ces résultats, bien qu'encourageants par rapport aux quantités d'interleukine-2 pouvant être obtenues après culture de lymphocytes périphériques sains ou de cellules lymphoblastoïdes, montraient que, contrairement à ce qu'il avait pu être constaté avec d'autres protéines telles que l'antigène de surface du virus de l'hépatite B ou l'hormone de croissance humaine, il n'était pas possible pour l'obtention d'interleukine-2 de tirer tout l'intérêt attendu de l'utilisation d'une séquence d'ADN codant pour la dhfr, accompagnée des moyens nécessaires à son expression.

Poursuivant ses investigations la demanderesse a constaté que, de façon très surprenante, le remplacement, sur les premiers vecteurs testés, au niveau de la séquence d'ADN codant pour le précurseur de l'interleukine-2 de la partie codant pour son peptide-signal par une séquence codant pour le peptide-signal de l'un des précurseurs naturels de l'hormone de croissance humaine (protéine ci-après désignée par l'abréviation hGH), permettait d'améliorer de manière significative le niveau de sécrétion et même, avec certaines constructions, de retrouver le niveau initial attendu. La demanderesse apporte ainsi une solution qui, satisfaisant aux critères qui lui sont afférents, permet d'envisager une production à l'échelle industrielle de l'interleukine-2.

Selon un premier aspect, l'invention concerne précisément des cellules eucaryotes recombinées productrices d'interleukine-2 qui contiennent, avec les moyens nécessaires à leur expression, une séquence d'ADN codant pour la dihydrofolate réductase et une séquence d'ADN codant pour un précurseur hybride de l'interleukine-2 dont le peptide-signal est celui de l'un des précurseurs naturels de l'hormone de croissance humaine.

Elle a également pour objet un procédé d'obtention desdites cellules consistant 1) à transfecter des cellules eucaryotes à l'aide d'un vecteur portant à la fois, avec les moyens nécessaires à leur expression, une séquence d'ADN codant pour la dihydrofolate réductase et une séquence d'ADN codant pour un précurseur hybride de l'interleukine-2 dont le peptide-signal est celui de l'un des précurseurs naturels de l'hormone de croissance humaine, puis 2) à sélectionner les cellules transfectées productrices d'interleukine-2 par culture dans des milieux successifs présentant l'un par rapport au précédent une concentration accrue en méthotrexate.

Enfin, elle a pour objet le procédé d'obtention d'interleukine-2 consistant à mettre en culture des cellules eucaryotes productrices d'interleukine-2, à recueillir le milieu de culture et à séparer l'interleukine-2 des autres constituants dudit milieu, procédé dans lequel les cellules mises en culture sont issues de cellules eucaryotes transfectées à l'aide d'un vecteur portant à la fois, avec les moyens nécessaires à leur expression, une séquence d'ADN codant pour la dihydrofolate réductase et une séquence d'ADN codant pour un précurseur hybride de l'interleukine-2 dont le peptide-signal est celui de l'un des précurseurs naturels de l'hormone de croissance humaine, puis sélectionnées par culture dans des milieux successifs présentant l'un par rapport au précédent une concentration accrue en méthotrexate.

Il doit être précisé que ledit procédé peut s'appliquer, non seulement et de manière particulièrement adaptée à l'obtention d'interleukine-2 d'origine humaine, mais également à l'obtention d'interleukine-2 d'autres origines animales, dans la mesure où de telles molécules pourraient présenter un intérêt particulier justifiant une application industrielle, par exemple en qualité d'agent de diagnostic ou de principe actif d'un médicament notamment à usage vétérinaire.

Il va de soi que ce procédé est destiné principalement à permettre l'obtention d'interleukine-2 glycosylée telle qu'elle est sécrétée par les lymphocytes-T naturellement producteurs. Il doit être noté que ce procédé convient également à l'obtention des formes incomplètement glycosylées ou non glycosylées de l'interleukine-2 présentes dans le milieu après culture des cellules eucaryotes selon l'invention.

Les cellules eucaryotes utiles pour la mise en oeuvre de l'invention sont des cellules d'origine animale capables de glycosylation. Parmi ces cellules, les cellules communément désignées par l'appellation CHO par référence à leur origine (cellules d'ovaire de hamster chinois) sont particulièrement adaptées.

Les techniques liées à l'utilisation de ces cellules, qu'il s'agisse de leur propagation ou de leur transfection par les vecteurs ou encore de la sélection des cellules effectivement transfectées, sont connues de l'homme de l'art. Certaines seront plus précisément décrites lors de la présentation des exemples.

Les vecteurs nécessaires pour la mise en oeuvre de l'invention peuvent revêtir diverses formes. Il peut s'agir de tout ou partie d'un génome viral et notamment du génome d'un rétrovirus ou d'un plasmide ou encore d'un cosmide. De façon avantageuse, un plasmide est mis en oeuvre.

Les vecteurs selon l'invention portent avec les moyens nécessaires à leur expression une séquence d'ADN codant pour la dihydrofolate réductase et une séquence d'ADN codant pour un précurseur hybride de l'interleukine-2 (précurseur ci-après symboliquement désigné par la notation : (ps-hGH)-interleukine-2) dont le peptide-signal est celui de l'un des précurseurs naturels de l'hGH.

La partie codante correspondant au peptide-signal peut avoir l'une des séquences que permet la dégénérescence du code génétique : un même acide aminé - hormis la méthionine - pouvant être codé par 2,3,4, ou même, pour certains, 6 codons. Une séquence de nucléotides préférée codant pour le peptide-signal ps-hGH est celle présentée à la figure 11 où a été noté, pour chacun des 26 codons, l'acide aminé lui correspondant.

Lesdites séquences d'ADN peuvent être incluses dans une même unité d'expression. D'une manière avantageuse chacune appartient à une unité d'expression autonome. Les moyens nécessaires à l'expression de ces séquences sont choisis parmi ceux généralement utilisés pour la construction de vecteurs destinés à la transfection de cellules eucaryotes. D'une manière préférée, ils sont issus du génome du virus SV 40 à partir duquel peuvent notamment être préparées des séquences d'ADN incluant en particulier le promoteur précoce, et/ou le signal précoce de polyadénylation (Fiers, W. (1978) Nature, 273, 113-120).

La construction des vecteurs selon l'invention fait appel à des techniques maintenant bien connues de l'homme de l'art.

Il doit être noté qu'en ce qui concerne la séquence d'ADN codant pour le précurseur (ps-hGH)-interleukine-2, il est intéressant de préparer un ADN complémentaire (en se référant par exemple aux travaux de Taniguchi, T. et al ((1983) Nature, 302, 305-310) ou encore à ceux de Devos. X. et al ((1983) Nucleic Acids Res., 11, 4307-4323) de l'ARN messager de lymphocytes-T codant pour le précurseur naturel de l'interleukine-2 puis de substituer à la séquence codant pour son peptide-signal une séquence codant pour le peptide-signal de l'un des précurseurs naturels de l'hGH.

Deux vecteurs préférés, construits en vue de l'obtention de l'interleukine-2 d'origine humaine sont les plasmides pSV 726 (figure 6) et pSV 741 (figure 9). Ils comportent chacun une unité d'expression pour la dhfr et une unité d'expression pour un précurseur (ps-hGH)-interleukine-2. Les deux plasmides diffèrent principalement par la présence, la nature et la position d'introns dans les unités d'expression. Plus précisément le plasmide pSV 726 porte un intron en aval de la séquence codant pour le précurseur (ps-hGH)-interleukine-2 ainsi qu'un intron en aval de la séquence codant pour la dhfr. Le plasmide pSV 741 porte par contre un intron en amont de la séquence codant pour le précurseur (ps-hGH)-interleukine-2 et ne présente aucun intron au niveau de son unité d'expression pour la dhfr.

Selon encore un autre aspect, l'invention concerne des lignées cellulaires sélectionnées, à partir des cellules eucaryotes ayant incorporé un vecteur selon l'invention, par culture desdites cellules dans des milieux successifs, présentant l'un par rapport au précédent une concentration accrue en méthotrexate. On poursuit les passages d'un milieu à l'autre jusqu'à ce que l'on ne constate plus une amplification de la sécrétion d'IL-2.

Des exemples de mise en oeuvre de l'invention sont présentés ci-après. Ils ne sont donnés bien entendu qu'à titre d'illustrations et ne sont en aucun cas limitatifs.

## EXEMPLES

Sont présentés ci-après à titre d'exemples deux vecteurs selon l'invention testés en conditions d'expression transitoire. L'obtention de lignées cellulaires hautement productrices puis la caractérisation de l'interleukine-2 d'origine humaine (ci-après désignée par l'abréviation IL-2) produite sont ensuite décrites.

### I. CONSTRUCTION ET TEST EN CONDITIONS D'EXPRESSION TRANSITOIRE DE DEUX VECTEURS: LES PLASMIDES PSV 726 ET PSV 741

#### 1. METHODES

A/ CONSTRUCTION DES VECTEURS

La construction des vecteurs comprend notamment l'isolement de fragments d'ADN à l'aide d'enzymes de restriction à partir de vecteurs préexistants, la synthèse par voie chimique d'oligonucléotides, l'assemblage - éventuellement après modification de leurs extrémités - de ces divers fragments en utilisant une enzyme telle que l'ADN - ligase du bactériophage T4, la sélection par clonage - après transformation bactérienne dans Escherichia coli - des vecteurs puis leur purification.

Elle fait appel à des techniques bien connues de l'homme de l'art.

Ces techniques sont décrites dans l'ouvrage intitulé Molecular Cloning : a Laboratory Manual de Maniatis, T; et al publié en 1982 par les Editions Cold Spring Harbor Press à New York (E.U.A.).

L'ensemble des enzymes de restriction nécessaires à la construction des vecteurs ci-après présentés est commercialisé notamment par la Société New England Biolabs (E.U.A.).

L'ADN-ligase du bactériophage T4 est disponible auprès de la Société New England Nuclear (E.U.A.).

La construction des vecteurs est explicitée à l'aide des figures 2 à 10 pour lesquelles la légende suivante a été adoptée :

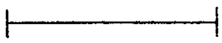

séquence d'ADN issue du plasmide pBR 322

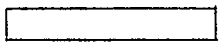

séquence d'ADN issue du génome du virus SV 40

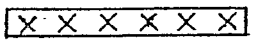

séquence d'ADN issue du gène codant pour l'alpha-globine de souris

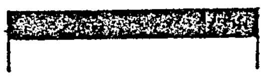


séquence d'ADN constituant la séquence codant pour le précurseur naturel de l'interleukine-2 humaine ou son variant dont un résidu alanine a été substitué au résidu tyrosine en position 2

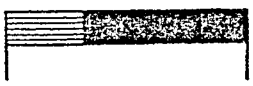


séquence d'ADN constituant la séquence codant pour le précurseur (ps-hGH)-IL-2

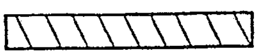

séquence d'ADN codant pour la dhfr.

B/ OBTENTION D'UNE SEQUENCE D'ADN CODANT POUR LE PRECURSEUR NATUREL DE L'INTERLEUKINE-2 D'ORIGINE HUMAINE

Un ADN complémentaire à l'ARN messager codant pour le précurseur de l'IL-2 isolé à partir de lymphocytes-T humains a été cloné.

La séquence nucléotidique, ainsi obtenue, les nucléotides étant regroupés par codons à la hauteur desquels on a porté les acides aminés du précurseur leur correspondant, est incluse dans la séquence d'ADN (brin 5'→3') présentée à la figure 1.

C/ MISE EN OEUVRE DES CELLULES EUCARYOTES

a. Choix.

Le choix s'est porté sur la souche DXB11 de cellules CHO DHFR- sélectionnée par Urlaub, G. et Chasin, L. ((1980) Proc. Natl. Acad. Sci. USA, 77, 4216-4220).

b. Mode opératoire pour l'expression transitoire.

Le protocole décrit par Sompayrac, L. et Danna, K. ((1981) Proc. Natl. Acad. Sci. USA, 78, 7575) a été mis en oeuvre.

Pour chaque essai : $5.10^5$ cellules sont ensemencées dans une boîte de Petri de 6 cm de diamètre contenant 5 ml de milieu alpha-MEM (Gibco, E.U.A.) additionné à raison de 5 % (v/v) de sérum foetal de veau (Gibco).

Après 24 heures d'incubation à 37° C, les cellules sont lavées avec 5 ml de tampon PBS (R. Dulbecco et M.

Vogt, J. Exp. Med. 99 (1954) 167) puis recouvertes de 1 ml de milieu alpha-MEM additionné de 0,05 M de tris (hydroxyméthyl) aminométhane - HCl (ou tris-HCl) à pH 7,3, de 0,2 mg de DEAE dextran de 500 000 daltons (Sigma, E.U.A.) et de 10 µg d'ADN plasmidique.

Et on procède à une incubation de 7 h à 37°C à l'issue de laquelle les cellules sont lavées avec 5 ml de tampon PBS puis recouvertes de 5 ml de milieu alpha-MEM additionné, à raison de 2 % (v/v) de sérum foetal de veau.

Les cellules sont alors placées en incubation à 37°C pendant 4 jours. Le milieu de culture est ensuite recueilli et sert à une mesure de l'activité de type IL-2.

D/ MESURE DE L'ACTIVITE DE TYPE IL-2

On mesure l'activité biologique des milieux de culture recueillis tels qu'au paragraphe C/b - selon le test colorimétrique de Mosmann, T. (1983) J. Immunol. Methods, 65, 55-63) - sur la prolifération de la lignée de lymphocytes-T de souris IL-2- dépendante CTLL-2 (Baker, P. et al (1979) J. Exp. Med., 149, 173). A titre de témoin, la préparation de référence décrite dans Lymphokine Research ((1984), 4, 193-227) est utilisée.

2. PLASMIDE PSV 726

A/ CONSTRUCTION

La construction du plasmide pSV 726 a pour point de départ le plasmide pSV 700 (figure 2).

Le plasmide pSV 700 résulte de l'assemblage de 5 fragments d'ADN :

- Un fragment PvuII - HindIII de 342 paires de bases (ci-après pb) issu du génome du virus SV 40 (Fiers, W. (1978) Nature, 273, 113-120), et contenant le promoteur précoce de ce virus.
- Un fragment HindIII - BamHI de 504 pb (figure 1) contenant une séquence d'ADN codant pour le précurseur naturel de l'IL-2 tel que synthétisé dans les lymphocytes humains.
- Un fragment BamHI - BalI de 305 pb issu du gène de l'alpha-globine de souris (Nishioka, Y. et Leder, P. (1979) Cell, 18, 875-882), et qui contient l'intron distal de ce gène.
- Un fragment HpaI - BamHI de 133 pb issu du génome du virus SV 40 et contenant le signal précoce de polyadénylation de ce virus.
- Un fragment BamHI - PvuII de 2672 pb issu du plasmide pBR 322 (Bolivar, F. (1977) Gene, 2, 95-113).

La séquence de nucléotides AGCTTCCACAATGTACAGG située à l'extrémité 5′ du brin codant du fragment HindIII - BamHI (figure 1) est ensuite remplacée par la séquence synthétique AGCTTCCACCATGGCTAGG de manière à disposer, au niveau des nucléotides encadrant le codon ATG, d'une séquence conforme à la séquence consensus CCACCATGG décrite par Kozak, M. ((1984) Nucleic Acids Res., 12, 857-872). Le plasmide pSV 703 est ainsi obtenu (figure 3).

Le segment d'ADN compris entre les sites de restriction HindIII et HgiAI situé dans la partie amont du segment HindIII - BamHI (dont le brin 5′ → 3′ est représenté sur la figure 12) du plasmide pSV 703 et contenant la séquence correspondant au peptide-signal modifié (il comporte un résidu alanine en position 2 au lieu du résidu tyrosine en raison de l'adoption d'une séquence conforme à la séquence consensus de Kozak) du précurseur naturel de l'IL-2 et au premier acide aminé de l'IL-2 mature, est ensuite remplacé par un oligonucléotide double-brin synthétique dont le brin codant 5′ → 3′ est représenté à la figure 11.

Cette séquence synthétique code à partir de son neuvième nucléotide pour le peptide-signal de l'un des précurseurs naturels de l'hGH (la séquence en acides aminés de ce peptide-signal est portée sur la figure 11, chaque acide aminé étant en regard du codon lui correspondant), ci-après noté peptide-signal de l'hGH, et pour le premier acide aminé de l'IL-2 mature.

Le plasmide obtenu est le plasmide pSV 706 (figure 4). Son segment HindIII-BamHI qui porte la séquence codant pour le précurseur (ps-hGH)-IL-2, est représenté sur la figure 13.

Enfin, le fragment compris entre les sites de restriction EcoRI et EcoRV de 185 pb du plasmide pSV 706 est remplacé par un fragment PvuII-EcoRI de 2677 pb issu du plasmide pSV2-dhfr (Subramani, S. et al (1981) Molecular and Cellular Biology, 1, 854-864) déposé à la collection ATCC sous la référence 37146. Le plasmide obtenu est le plasmide pSV 726 (figure 6).

Le plasmide pSV 726 contient :

- Une unité d'expression pour le précurseur (ps-hGH)-IL-2. Cette unité a pour promoteur le promoteur précoce du virus SV 40 ; elle comporte, en aval de la séquence codant pour le précurseur de (ps-hGH)-IL-2, une séquence qui contient le deuxième intron du gène de l'alpha-globine de souris puis le signal précoce de polyadénylation du virus SV 40.
- Une unité d'expression pour la dhfr. Cette unité a pour promoteur le promoteur précoce du virus SV 40 ; elle comporte, en aval de la séquence codant pour la dhfr, la séquence comprise entre les sites MboI en positions 4693 et 4083 - selon la notation de Fiers, W. - sur le génome du virus SV 40 et contenant un intron pour l'antigène t du virus SV 40 puis le signal précoce de polyadénylation du virus SV 40. Cette unité est contenue dans le fragment PvuII-EcoRI issu du plasmide pSV2-dhfr.

B/ AVANTAGES LIES A L'UTILISATION DU PLASMIDE PSV 726

Un essai comparatif a été réalisé.

Les plasmides pSV 703, pSV 720 (présenté ci-après) et pSV 726 ont été testés dans des conditions d'expression transitoire (cf méthodes). On a mesuré l'activité (selon le protocole indiqué plus haut) de type IL-2 de chaque surnageant de culture de manière à apprécier le niveau de sécrétion de l'IL-2 dont sont capables les cellules transfectées avec chacun des plasmides.

Le plasmide pSV 720 (figure 5) est un dérivé du plasmide pSV 703. Il a été obtenu en substituant au fragment compris entre les sites de restriction EcoRI et EcoRV de 185 pb du plasmide pSV 703 un fragment PvuII - EcoRI de 2677 pb issu du plasmide pSV2 - dhfr.

Les plasmides pSV 720 et pSV 726 portent de ce fait la même unité d'expression pour la dhfr. Ils ne diffèrent que par leur séquence d'ADN respective codant pour le peptide-signal du précurseur de l'IL-2.

Cette séquence code dans le cas du plasmide pSV 720, pour une variante du peptide-signal du précurseur naturel de l'IL-2 et dans le cas du plasmide pSV 726 pour le peptide-signal de l'hGH.

Le tableau 1 ci-après présente les résultats de cet essai:

TABLEAU 1

| PLASMIDE | ACTIVITE IL-2 (U/ml) |
|---|---|
| pSV 703 | 228 ± 112 |
| pSV 720 | 34 ± 29 |
| pSV 726 | 153 ± 68 |

Ce tableau montre la chute de sécrétion - en conditions d'expression transitoire - liée à l'introduction dans un plasmide (pSV 703) d'une unité d'expression pour la dhfr (plasmide pSV 720). Il indique de manière nette que la substitution à la séquence codant pour le variant du peptide-signal du précurseur naturel de l'IL-2 de la séquence codant pour le peptide-signal de l'hGH (plasmide pSV 726) permet d'améliorer de manière significative le niveau de sécrétion et même de retrouver un niveau sensiblement équivalent à celui déterminé pour le plasmide d'origine (plasmide pSV 703).

3. PLASMIDE PSV 741

A/ CONSTRUCTION

La construction du plasmide pSV 741 a pour point de départ le plasmide pSV 739 (figure 7).

Le plasmide pSV 739 résulte de l'assemblage de 5 fragments d'ADN :

- Un fragment EcoRV-BglI de 777 pb issu du génome du virus SV 40 et contenant une partie du promoteur précoce du virus SV 40.
- un fragment BglI-PstI de 239 pb issu du plasmide pl1 (Okayama, H. et Berg, P. (1983) Molecular and Cellular Biology, 3, 280-289) et contenant la partie manquante dans le fragment EcoRV-BglI du promoteur précoce du virus SV 40 et les 2 introns de l'ARN messager tardif 19 S de la protéine VP2 et de l'ARN messager tardif 16 S de la protéine VP1 (Fiers, W. (1978) Nature 273, 113-120).
- Un fragment PstI-BamHI de 525 pb constitué du fragment HindIII-BamHI de 521 pb du plasmide pSV 706 (cf figure 13) allongé d'un linker PstI-HindIII ; ce fragment contient la séquence d'ADN codant pour le précurseur (ps-hGH)-IL-2.
- Un fragment BclI-EcoRI de 988 pb issu du génome du virus SV 40 et contenant le signal précoce de polyadénylation de ce virus.
- Un fragment EcoRI-PvuII de 2295 pb issu du plasmide pBR 322.

Le plasmide pSV 741 (figure 9) est obtenu en substituant au fragment BamHI-EcoRI du plasmide pSV 739 un fragment PvuII-EcoRI résultant lui-même de l'assemblage d'un fragment BclI-EcoRI de 988 pb issu du génome du virus SV 40 et contenant le signal précoce de polyadénylation de ce virus et d'un fragment PvuII-BglII de 1103 pb issu du plasmide pSV2-dhfr.

Le plasmide pSV 741 contient :

- Une unité d'expression pour le précurseur (ps-hGH)-IL-2. Cette unité a pour promoteur le promoteur précoce du virus SV 40. Elle comporte , en amont de la séquence codant pour le précurseur de l'IL-2, une séquence constituée de deux introns du virus SV 40 et, en aval de cette séquence, le signal précoce de polyadénylation du virus SV 40.
- Une unité d'expression pour la dhfr. Cette unité comporte le promoteur précoce du virus SV 40, une séquence d'ADN codant pour la dhfr et, en aval de cette séquence, sans intron intermédiaire, le signal précoce de polyadénylation du virus SV 40.

B/ AVANTAGES LIES A L'UTILISATION DU PLASMIDE PSV 741

Un essai comparatif a été réalisé.

Les plasmides pSV 739, pSV 741 et pSV 742 (cf ci-après) ont été testés dans des conditions d'expression transitoire (cf méthodes).

On a mesuré (selon le protocole indiqué plus haut) l'activité de type IL-2 de chaque milieu de culture de manière à apprécier le niveau de sécrétion de l'IL-2 dont sont capables les cellules transfectées avec chacun des plasmides.

Le plasmide pSV 742 (figure 10) a été construit à partir du plasmide pSV 739.

On a remplacé le fragment HindIII-XbaI de 260 pb du plasmide PSV 739 (figure 13) par le fragment HindIII-XbaI de 244 pb (figure 12) du plasmide pSV 703 (figure 3). Le plasmide obtenu est le plasmide pSV 740 (figure 8).

Le plasmide pSV 742 a été obtenu en substituant au fragment EcoRI-BamHI du plasmide pSV 740 un fragment PvuII-EcoRI résultant lui-même de l'assemblage d'un fragment BglII-PvuII de 1103 pb issu du plasmide pSV2-dhfr et d'un fragment EcoRI-BclI de 988 pb issu du génome du virus SV 40 et contenant le signal précoce de polyadénylation de ce virus.

Les plasmides pSV 741 et pSV 742 portent de ce fait la même unité d'expression pour la dhfr. Ils ne diffèrent que par la composition de leur séquence d'ADN respective codant pour le peptide-signal du précurseur de l'IL-2. Cette séquence code dans le cas du plasmide PSV 742 pour une variante du peptide-signal du précurseur naturel de l'IL-2 et dans le cas du plasmide pSV 741 pour le peptide-signal de l'hGH.

Le tableau 2 ci-après présente les résultats de cet essai :

TABLEAU 2

| PLASMIDE | ACTIVITE IL-2 (U/ml) |
|---|---|
| pSV 740 | 171 ± 10 |
| pSV 741 | 46 ± 4 |
| pSV 742 | 7 ± 2 |

Ce tableau montre la chute de sécrétion - en conditions d'expression transitoire - liée à l'introduction dans le plasmide PSV 740 d'une unité d'expression pour la dhfr (plasmide pSV 742). Il indique de manière nette que la substitution de la séquence codant pour un variant du peptide-signal du précurseur naturel de l'Il-2 par une séquence codant pour le peptide-signal de l'hGH (plasmide pSV 741) permet d'améliorer de manière significative le niveau de sécrétion d'IL-2.

II. OBTENTION DE LIGNEES CELLULAIRES HAUTEMENT PRODUCTRICES.

Des cellules CHO DHFR- de la souche DXB11 ont été transfectées avec l'un ou l'autre des plasmides pSV 726 et pSV 741.

Le mode opératoire décrit par Graham, F. et Van der Eb, A. ((1973) Virology, 54, 536-539) a été suivi.

Les cellules sont tout d'abord propagées dans du milieu alpha-MEM (Gibco) additionné, à raison de 10 % (v/v) de sérum foetal de veau, de 20 µg/ml de gentamycine, de 60 µg/ml de tylocine et de 300 µg/ml de L-glutamine (ci-après milieu non sélectif).

Après une phase de lavage, les cellules ensemencées la veille - à raison de 0,8 $10^6$ pour une boîte de Petri de 10 cm de diamètre - sont recouvertes de milieu non sélectif et l'on ajoute 10 µg de l'un des plasmides en présence de phosphate de calcium mais sans ADN de sperme de saumon. Des cellules ainsi préparées sont incubées 7 heures à 37° C.

Les cellules sont ensuite cultivées dans du milieu alpha-MEM additionné, à raison de 5 % (v/v), de sérum foetal de veau pendant 3 jours à 37° C. A l'issue de cette incubation, elles sont réparties à raison de $5.10^5$ cellules par boîte, dans des boîtes de Petri contenant du milieu qui, constitué de milieu essentiel minimum contenant en outre des sels, est commercialisé par la Société Gibco sous la référence 041-1095 ; ce milieu est ici utilisé additionné de sérum foetal de veau dialysé Gibco (10 % v/v), de gentamycine (20 µg/ml), de tylocine (50 µg/ml), de L-glutamine (300 µg/ml) et de L-proline (150 µg/ml). Ce milieu ainsi complété constitue le milieu sélectif cité ci-après.

Les cellules ainsi préparées sont incubées à 37° C pendant 2 semaines en renouvelant le milieu sélectif tous les 3 jours. Les colonies observées à l'issue de cette incubation sont en principe issues de cellules ayant effectivement incorporé un plasmide. Ces colonies sont prélevées et remises en culture séparément dans du milieu sélectif et testées par une mesure de l'activité de type IL-2 pour s'assurer de leur aptitude à produire de l'IL-2.

C'est ainsi qu'après transfection ont pu être isolées et se sont révélées positives 347 colonies avec le plasmide pSV 726.

Les colonies les plus productrices (35 000 à 50 000 unités d'IL-2/ml mesurées au bout de 4 jours, en partant d'une population initiale de $4.10^5$ cellules) ont été mises en culture. Les cellules ont été repiquées successivement dans 4 préparations de milieu sélectif présentant l'une par rapport à la précédente une concentration accrue (0,02, 0,05, 0,1 puis 0,2 µM) en méthotrexate (amethopterin, Sigma) de la manière décrite par Alt. F. et al ((1978) Journal of Biological Chemistry, 253, 1357-1570).

A l'issue de cette phase opératoire, plusieurs lignées hautement productrices ont pu être sélectionnées.

C'est ainsi que la lignée 109.12 transfectée avec le plasmide pSV 726 est capable d'un niveau de sécrétion d'IL-2, après 4 jours de culture, exprimé en terme d'activité, de 250 000 unités/ml.

III. CARACTERISATION DE L'IL-2 SECRETEE PAR LES LIGNEES

La mise en culture à plus grande échelle des lignées hautement productrices décrites au paragraphe II a permis de disposer, par traitement des surnageants de culture, après séparation des autres constituants, de la protéine sécrétée par les cellules en quantité suffisante pour en permettre la caractérisation.

1. PURIFICATION DE L'IL-2

L'IL-2 est purifiée à partir d'un litre de surnageant de culture.

On procède tout d'abord à une concentration et à une première purification en soumettant le surnageant à une chromatographie d'échange d'ions sur une colonne d'agarose Sepharose[R] (S - Fast Flow - Pharmacia Fine Chemical - Suède) préalablement équilibrée avec de l'acétate d'ammonium 0,05 M à pH 4,5. L'élution est réalisée à l'aide d'acétate d'ammonium 0,05 M (pH 5,5) additionné de NaCl de molarité 0,05 M puis 0,5 M.

Les fractions éluées, qui s'avèrent biologiquement actives d'après une mesure de leur activité de type IL-2, sont rassemblées et leur pool est soumis à une chromatographie liquide à haute pression sur une colonne de phases inversées. Le support choisi est un gel de silice greffée en $C_3$. La colonne a pour dimensions : 1,0 x 25,0 cm.

L'élution est réalisée, avec un gradient linéaire d'acétonitrile variant de 5 à 100 % (v/v) dans une solution dans l'eau à 0,1 % (v/v) d'acide trifluoroacétique, en 80 min avec un débit de 4 ml/min.

Les fractions éluées, biologiquement actives, sont rassemblées et leur pool est soumis à une chromatographie de même type que celle ci-dessus réalisée, dans des conditions notamment d'élution identiques, sur un gel de silice greffée en $C_{18}$ dans une colonne de dimensions : 2,1 x 10,0 cm.

Le pool des fractions éluées recueillies lors de cette chromatographie, biologiquement actives, et présentant, d'après les résultats d'une électrophorèse sur gel de polyacrylamide en présence de dodécylsulfate de sodium (Laemli, (1970) Nature, 277, 680-685), une pureté en IL-2 supérieure à 95 % constitue le matériel sur lequel est effectuée la caractérisation de l'IL-2.

2. CARACTERISATION DE L'IL-2 PAR DETERMINATION DE LA SEQUENCE AMINO-TERMINALE

Les échantillons à traiter sont portés à la surface d'un filtre de bromure d'hexadiméthrine (ou polybrene). Le filtre est introduit dans un séquenceur de protéines (modèle 470 A commercialisé par la Société Applied Biosystems (E.U.A.)) équipé d'un chromatographe (Modèle 130A - Applied Biosystems) qui analyse in fine les dérivés phénylthiohydantoïques formés.

Les résultats de cette détermination sont en accord avec la séquence déjà publiée pour le produit naturel (Robb, R. et al (1984) Proc. Natl. Acad. Sci. U.S.A., 81, 6486-6490).

Cette séquence s'écrit pour ses dix premiers acides aminés :

```
1                                     10
Ala-Pro-Thr-Ser-Ser-Ser-Thr-Lys-Lys-Thr
```

L'alanine est le seul résidu détecté en position N-terminale. Cela apporte la confirmation que le précurseur (ps-hGH)-Il-2 a été correctement coupé lors de la sécrétion.

En conclusion, ces exemples mettent bien en évidence l'intérêt de l'invention qui permet une utilisation probante pour la production d'interleukine-2 de cellules eucaryotes en mettant à profit les qualités propres au système de sélection et/ou d'amplification se fondant sur la transfection des cellules par un vecteur portant, avec les moyens nécessaires à son expression, une séquence codant pour la dihydrofolate-réductase.

## Revendications

1. Cellules eucaryotes productrices d'interleukine-2, caractérisées en ce qu'elles contiennent, avec les moyens nécessaires à leur expression, une séquence d'ADN codant pour la dihydrofolate réductase et une séquence d'ADN codant pour un précurseur hybride de l'interleukine-2 dont le peptide-signal est celui de l'un des précurseurs naturels de l'hormone de croissance humaine.

2. Cellules eucaryotes selon la revendication 1, caractérisées en ce que la séquence d'ADN codant pour le précurseur hybride est la séquence d'ADN codant pour l'interleukine-2 d'origine humaine dont le peptide-signal est celui de l'un des précurseurs naturels de l'hormone de croissance humaine.

3. Cellules eucaryotes selon l'une des revendications 1 ou 2, caractérisées en ce qu'elles sont des cellules CHO.

4. Procédé d'obtention des cellules eucaryotes productrices d'interleukine-2 selon l'une quelconque des revendications 1 à 3, caractérisé en ce qu'il consiste à transfecter des cellules eucaryotes à l'aide d'un vecteur portant à la fois, avec les moyens nécessaires à leur expression, une séquence d'ADN codant pour la dihydrofolate réductase et une séquence d'ADN codant pour un précurseur hybride de l'interleukine-2 dont le peptide-signal est celui de l'un des précurseurs naturels de l'hormone de croissance humaine, puis à sélectionner ensuite les cellules transfectées productrices d'interleukine-2 par culture dans des milieux successifs présentant l'un par rapport au précédent une concentration accrue en méthotrexate.

5. Procédé selon la revendication 4, caractérisé en ce que les cellules eucaryotes sont des cellules CHO.

6. Procédé selon l'une des revendications 4 ou 5, caractérisé en ce que le vecteur porte une seule unité d'expression pour la dihydrofolate réductase et le précurseur de l'interleukine-2.

7. Procédé selon l'une des revendications 4 ou 5, caractérisé en ce que le vecteur porte deux unités d'expression distinctes, l'une pour la dihydrofolate réductase et l'autre pour le précurseur de l'interleukine-2.

8. Procédé selon l'une quelconque des revendications 4 à 7, caractérisé en ce que le précurseur hybride est la séquence d'ADN codant pour l'interleukine-2 d'origine humaine dont le peptide-signal est celui de l'un des précurseurs naturels de l'hormone de croissance humaine.

9. Procédé selon l'une quelconque des revendications 4 à 8, caractérisé en ce que le vecteur a les caractéristiques de l'un des plasmides pSV 726 et pSV 741.

10. Vecteur d'expression caractérisé en ce qu'il porte, avec les moyens nécessaires à leur expression, une séquence d'ADN codant pour la dihydrofolate réductase et une séquence d'ADN codant pour un précurseur hybride de l'interleukine-2 dont le peptide-signal est celui de l'un des précurseurs naturels de l'hormone de croissance humaine.

11. Vecteur selon la revendication 10, caractérisé en ce qu'il porte une seule unité d'expression pour la dihydrofolate réductase et le précurseur de l'interleukine-2.

12. Vecteur selon la revendication 10, caractérisé en ce qu'il porte deux unités d'expression distinctes, l'une pour la dihydrofolate réductase et l'autre pour le précurseur de l'interleukine-2.

13. Vecteur selon l'une quelconque des revendications 10 à 12, caractérisé en ce qu'il a les caractéristiques de l'un des plasmides pSV 726 et pSV 741.

14. Procédé d'obtention d'interleukine-2 consistant à mettre en culture des cellules eucaryotes productrices d'interleukine-2, à recueillir le milieu de culture et à séparer des autres constituants l'interleukine-2 contenue dans ledit milieu, caractérisé en ce que les cellules mises en culture sont issues de cellules eucaryotes transfectées à l'aide d'un vecteur portant à la fois, avec les moyens nécessaires à leur expression, une séquence d'ADN codant pour la dihydrofolate réductase et une séquence d'ADN codant pour un précurseur hybride de l'interleukine-2 dont le peptide-signal est celui de l'un des précurseurs naturels de l'hormone de croissance humaine, puis sélectionnées par culture dans des milieux successifs présentant l'un par rapport au précédent une concentration accrue en méthotrexate.

15. Interleukine-2 obtenue par le procédé selon la revendication 14.

FIG.1

```
                  -20
                  Met Tyr Arg Met Gln Leu Leu Ser Cys Ile Ala
5'  AGCTTCCACA    ATG TAC AGG ATG CAA CTC CTG TCT TGC ATT GCA
                                          -1   1
Leu Ser Leu Ala Leu Val Thr Asn Ser Ala Pro Thr Ser Ser Ser
CTA AGT CTT GCA CTT GTC ACA AAC AGT GCA CCT ACT TCA AGT TCT

Thr Lys Lys Thr Gln Leu Gln Leu Glu His Leu Leu Leu Asp Leu
ACA AAG AAA ACA CAG CTA CAA CTG GAG CAT TTA CTT CTG GAT TTA

Gln Met Ile Leu Asn Gly Ile Asn Asn Tyr Lys Asn Pro Lys Leu
CAG ATG ATT TTG AAT GGA ATT AAT AAT TAC AAG AAT CCC AAA CTC

Thr Arg Met Leu Thr Phe Lys Phe Tyr Met Pro Lys Lys Ala Thr
ACC AGG ATG CTC ACA TTT AAG TTT TAC ATG CCC AAG AAG GCC ACA

Glu Leu Lys His Leu Gln Cys Leu Glu Glu Glu Leu Lys Pro Leu
GAA CTG AAA CAT CTT CAG TGT CTA GAA GAA GAA CTC AAA CCT CTG

Glu Glu Val Leu Asn Leu Ala Gln Ser Lys Asn Phe His Leu Arg
GAG GAA GTG CTA AAT TTA GCT CAA AGC AAA AAC TTT CAC TTA AGA

Pro Arg Asp Leu Ile Ser Asn Ile Asn Val Ile Val Leu Glu Leu
CCC AGG GAC TTA ATC AGC AAT ATC AAC GTA ATA GTT CTG GAA CTA

Lys Gly Ser Glu Thr Thr Phe Met Cys Glu Tyr Ala Asp Glu Thr
AAG GGA TCT GAA ACA ACA TTC ATG TGT GAA TAT GCT GAT GAG ACA

Ala Thr Ile Val Glu Phe Leu Asn Arg Trp Ile Thr Phe Cys Gln
GCA ACC ATT GTA GAA TTT CTG AAC AGA TGG ATT ACC TTT TGT CAA

                        133
Ser Ile Ile Ser Thr Leu Thr
AGC ATC ATC TCA ACA CTG ACT TGA TAATTAAGTGCTTCCCACTTAAAACATATCAG 3'
```

FIG. 2

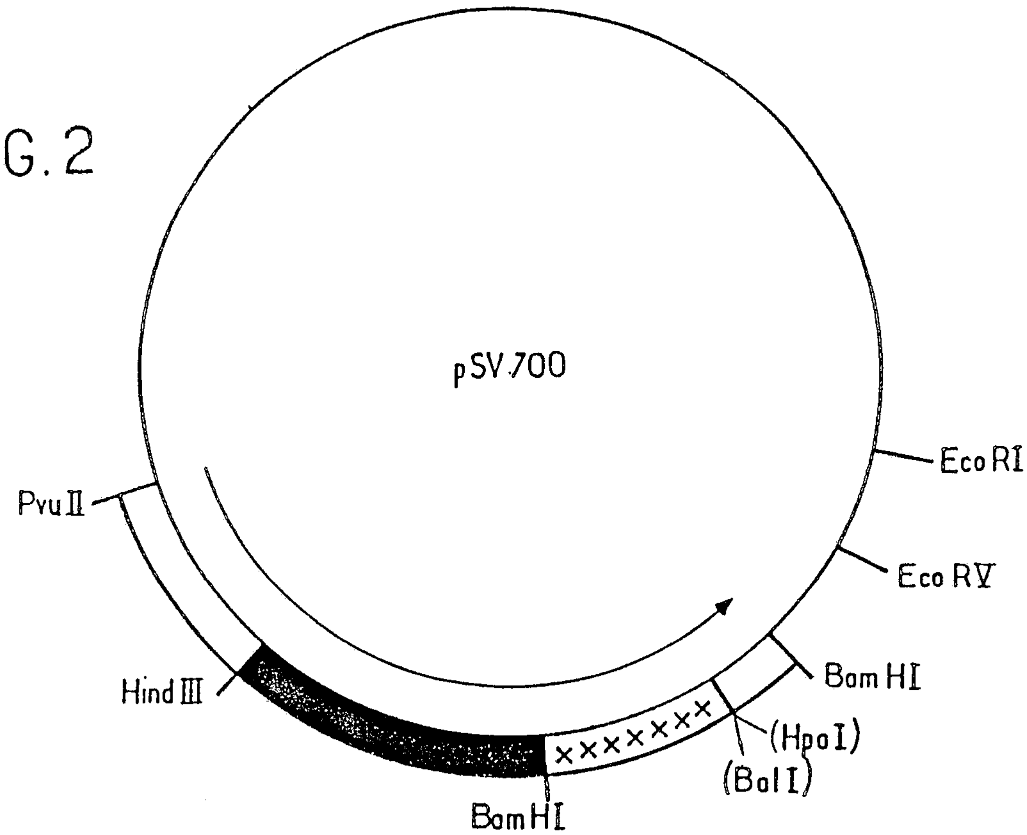

FIG.3

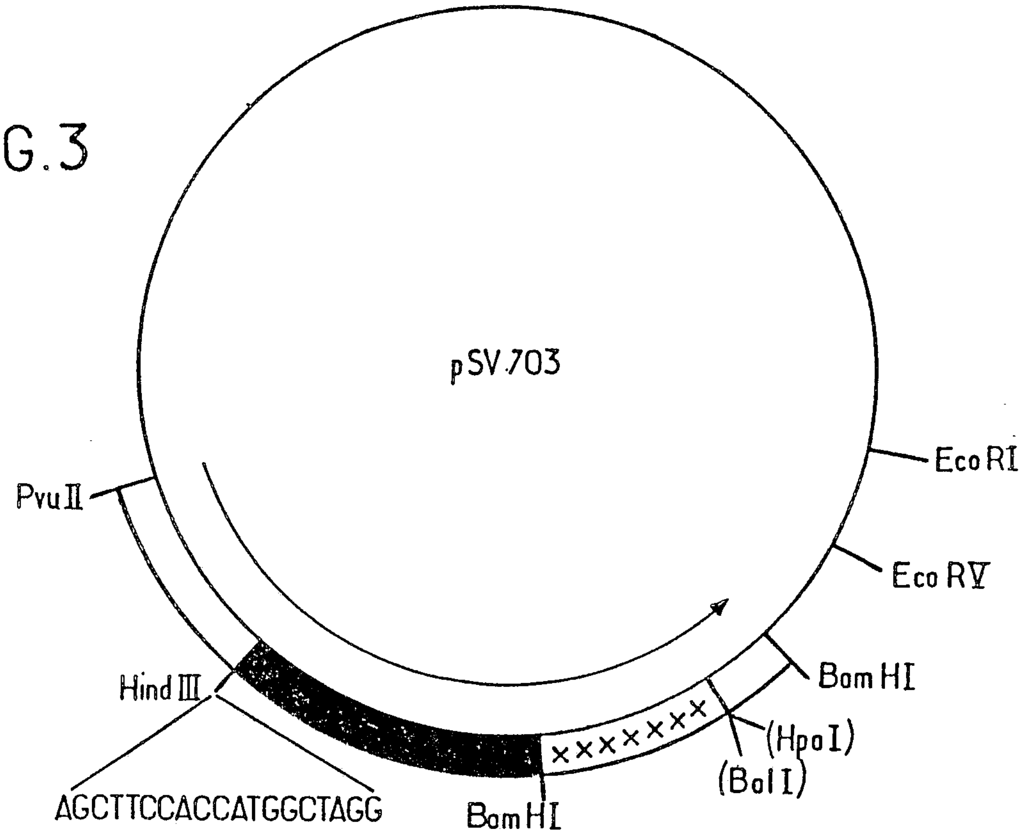
pSV 703
EcoRI
EcoRV
BamHI
(HpaI)
(BalI)
BamHI
PvuII
HindIII
AGCTTCCACCATGGCTAGG

FIG.5

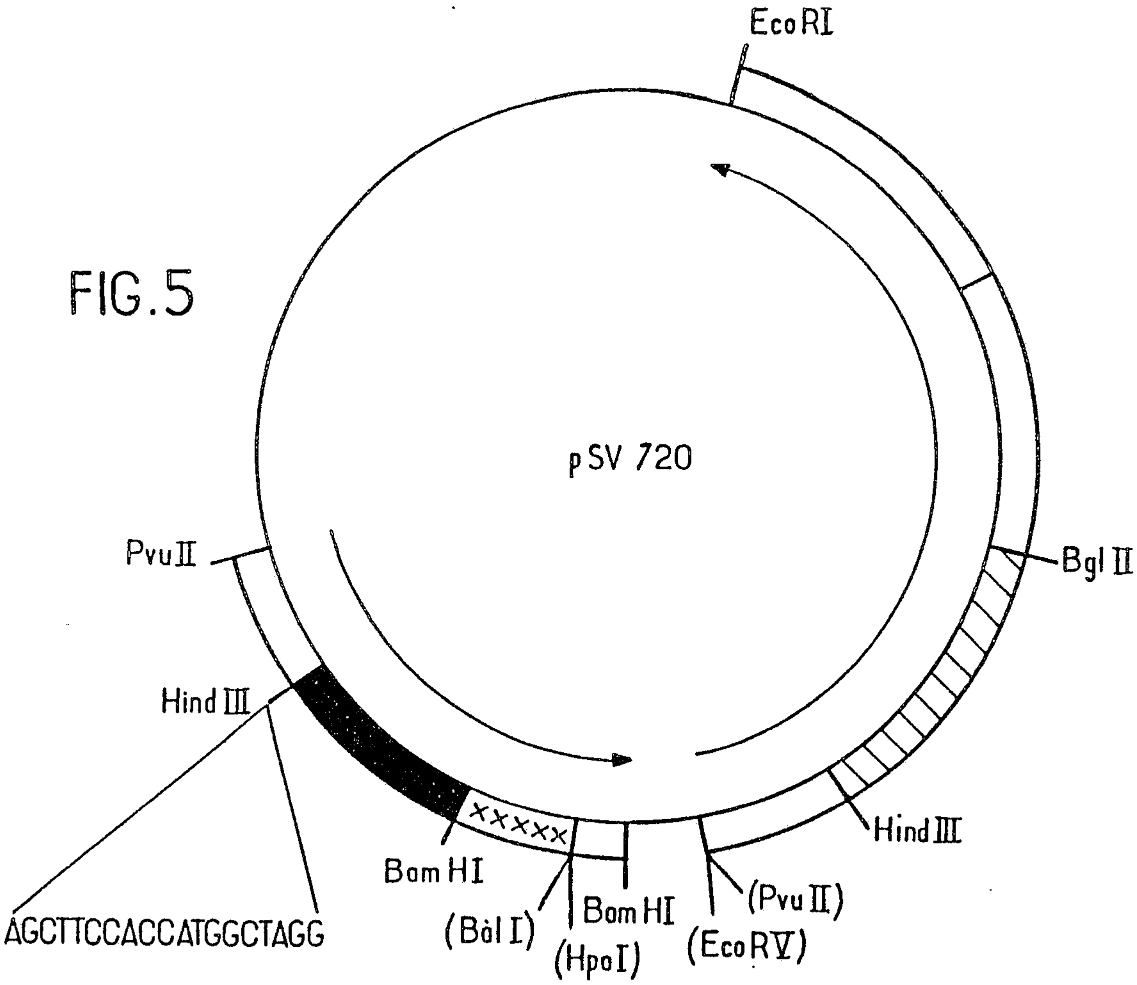
EcoRI
pSV 720
PvuII
BglII
HindIII
AGCTTCCACCATGGCTAGG
BamHI
(BalI)
BamHI
(HpaI)
(EcoRV)
(PvuII)
HindIII

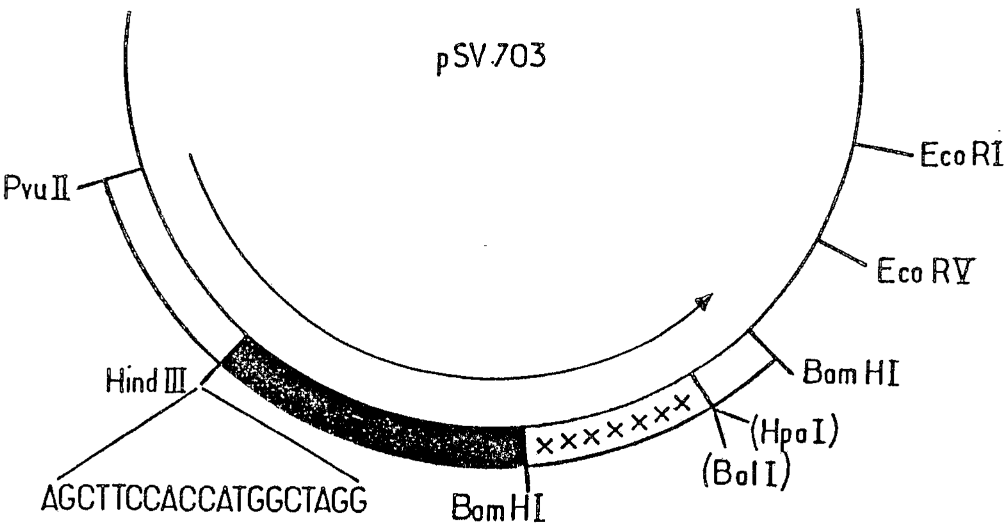
pSV 703
EcoRI
EcoRV
BamHI
(HpaI)
(BalI)
BamHI
PvuII
HindIII
AGCTTCCACCATGGCTAGG

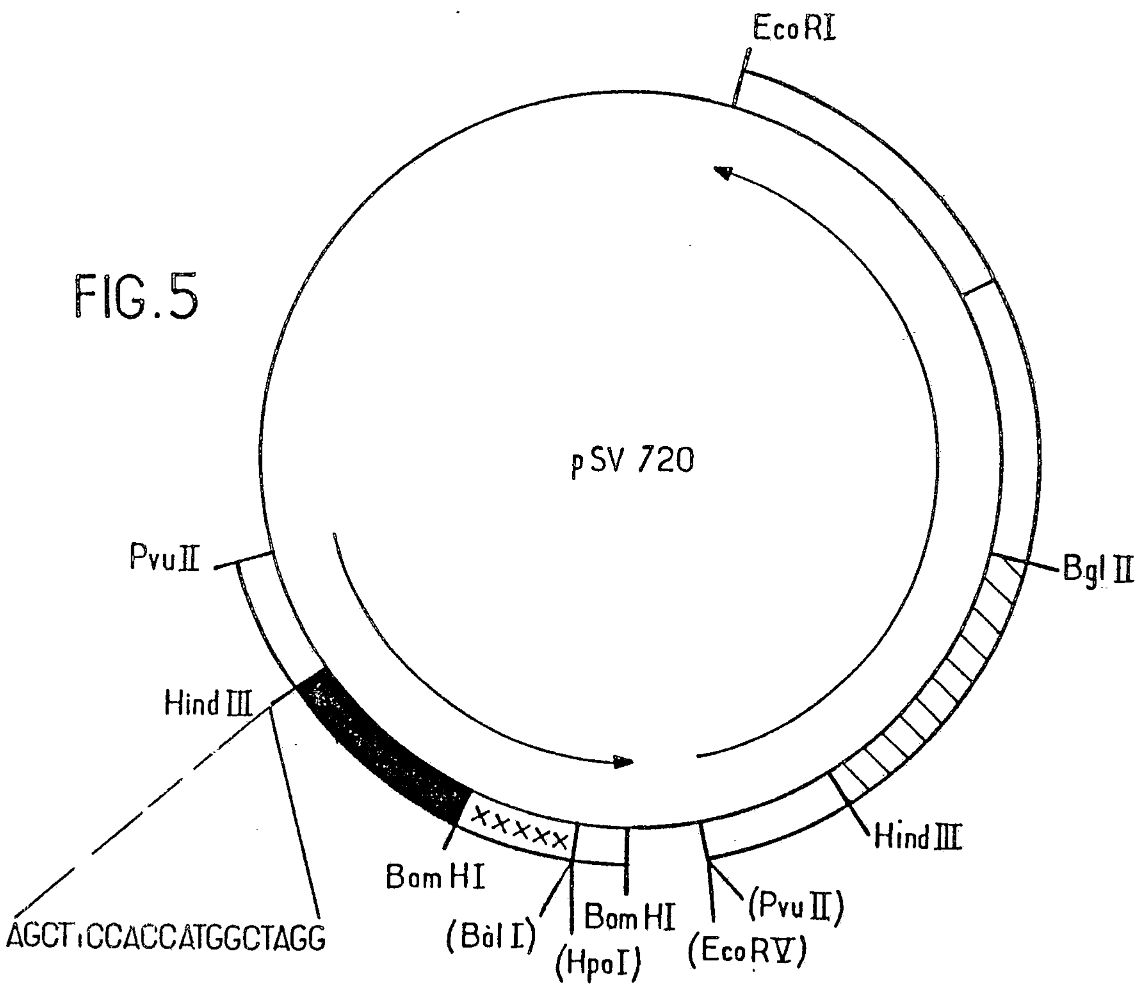
EcoRI
pSV 720
PvuII
HindIII
BglII
HindIII
AGCT CCACCATGGCTAGG
BamHI
(BalI)
BamHI
(HpaI)
(EcoRV)
(PvuII)

FIG.5

FIG. 4

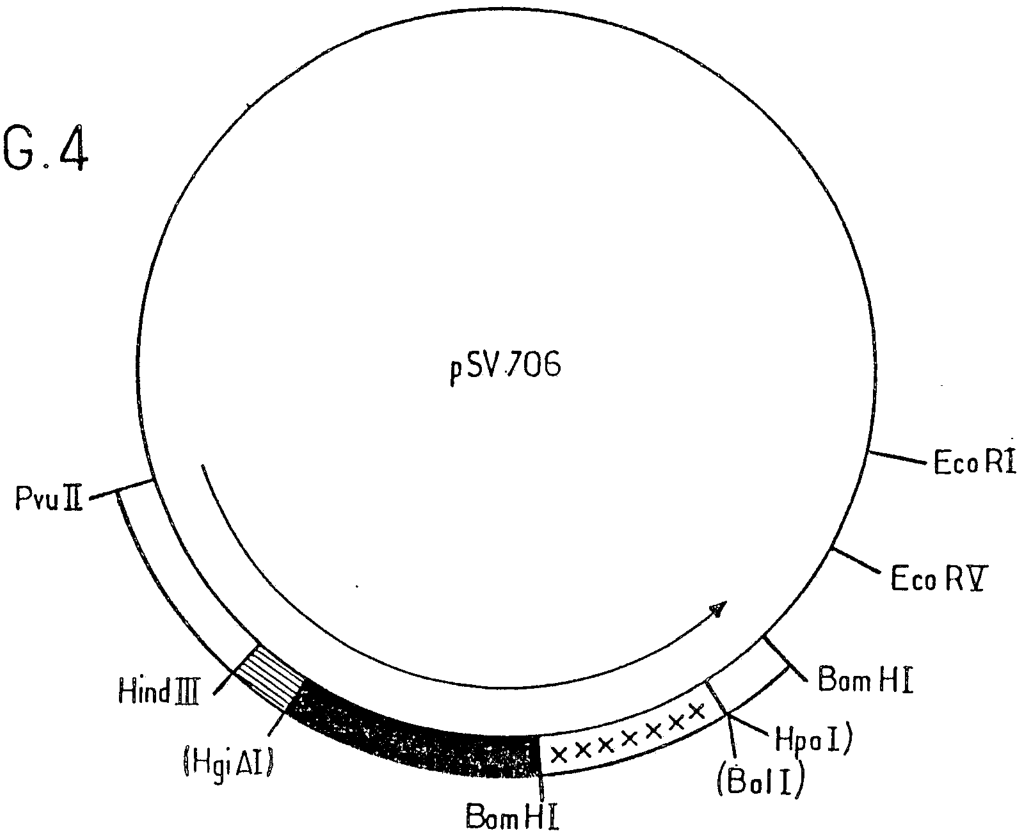
pSV.706
EcoRI
EcoRV
BamHI
HpaI)
(BalI)
BamHI
(HgiAI)
HindIII
PvuII

FIG. 6

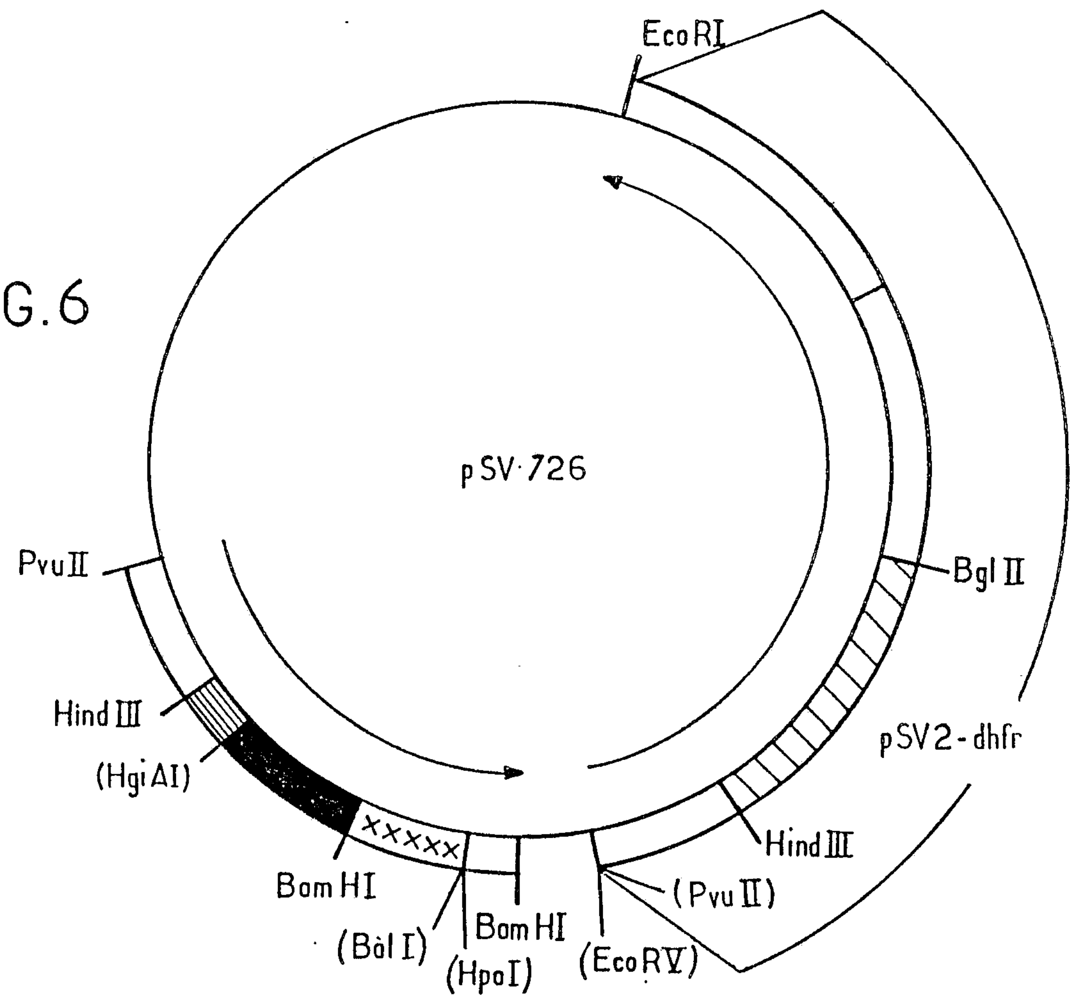
EcoRI
pSV.726
PvuII
BglII
HindIII
(HgiAI)
pSV2-dhfr
HindIII
BamHI
(BalI)
BamHI
(HpaI)
(EcoRV)
(PvuII)

FIG. 7

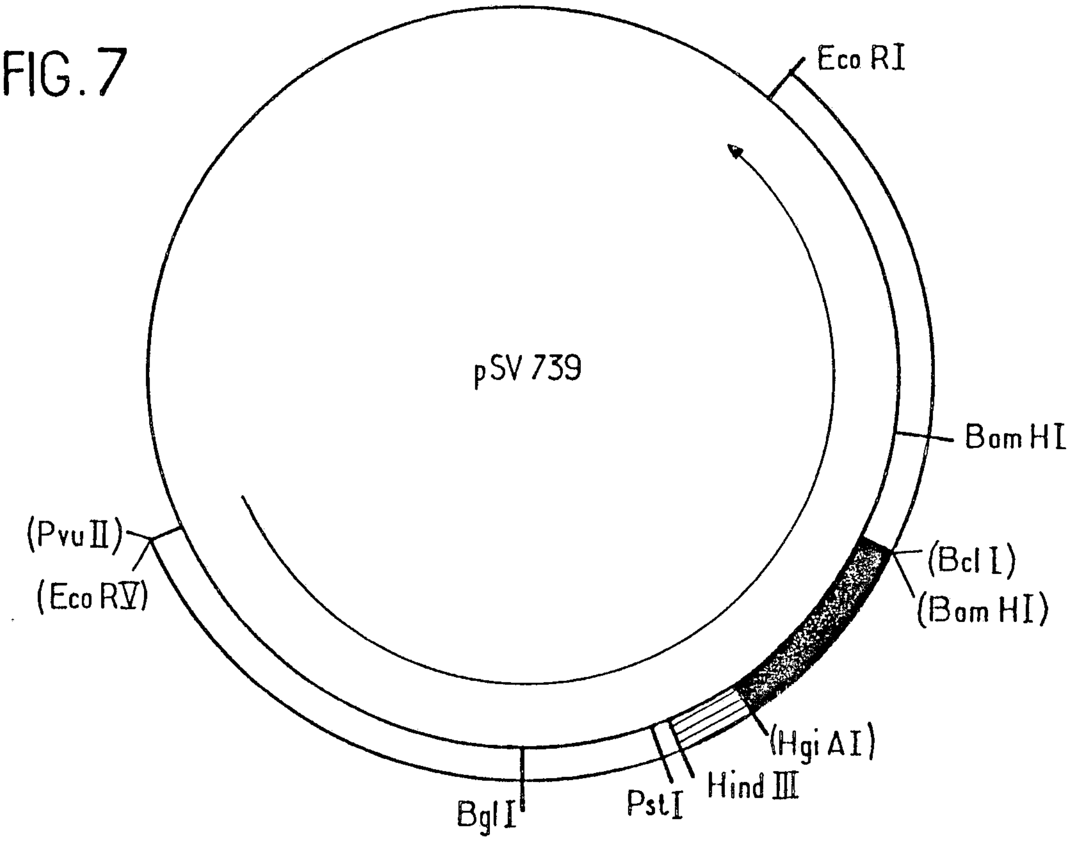
Eco RI
pSV 739
Bam HI
(Bcl I)
(Bam HI)
(Hgi AI)
Hind III
Pst I
Bgl I
(Pvu II)
(Eco RV)

FIG. 9

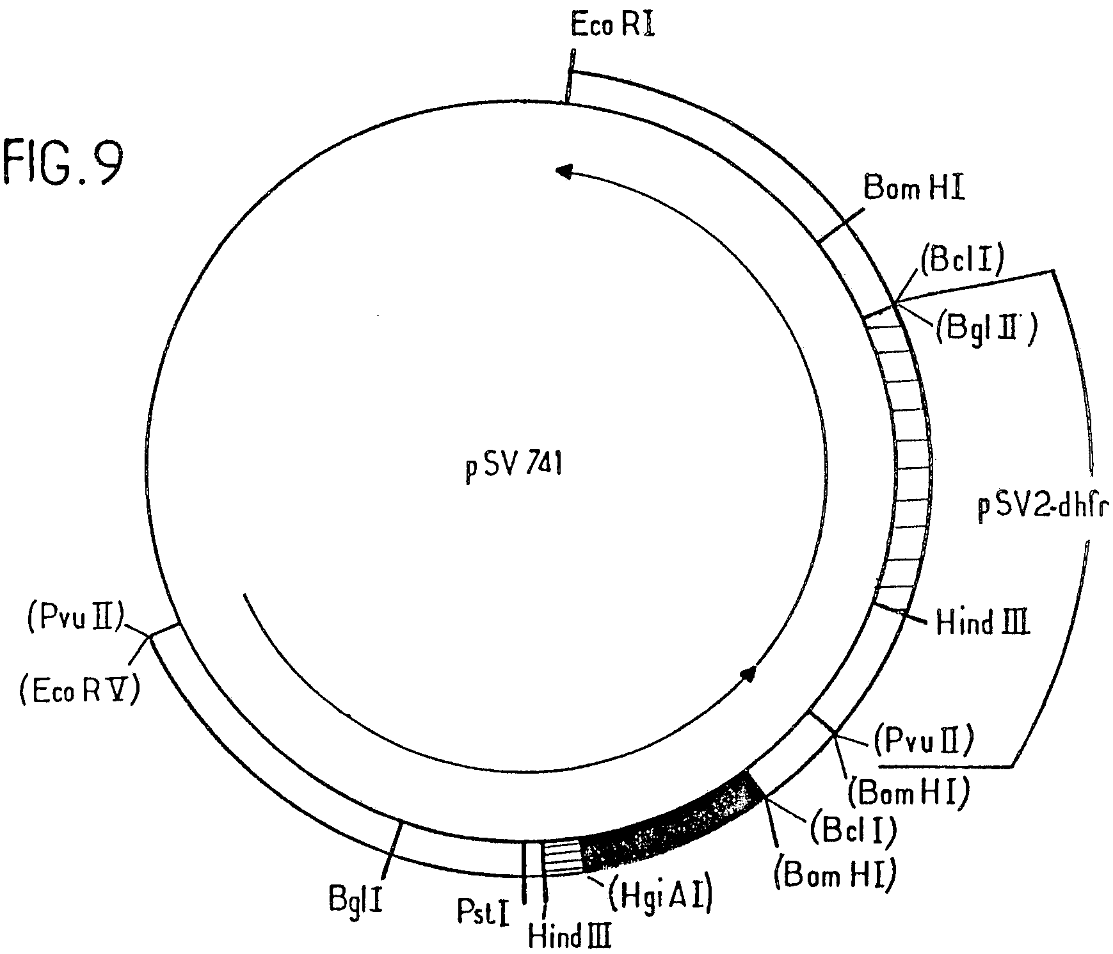
Eco RI
Bam HI
(Bcl I)
(Bgl II)
pSV 741
pSV2-dhfr
Hind III
(Pvu II)
(Bam HI)
(Bcl I)
(Bam HI)
(Hgi AI)
Hind III
Pst I
Bgl I
(Pvu II)
(Eco RV)

FIG. 8

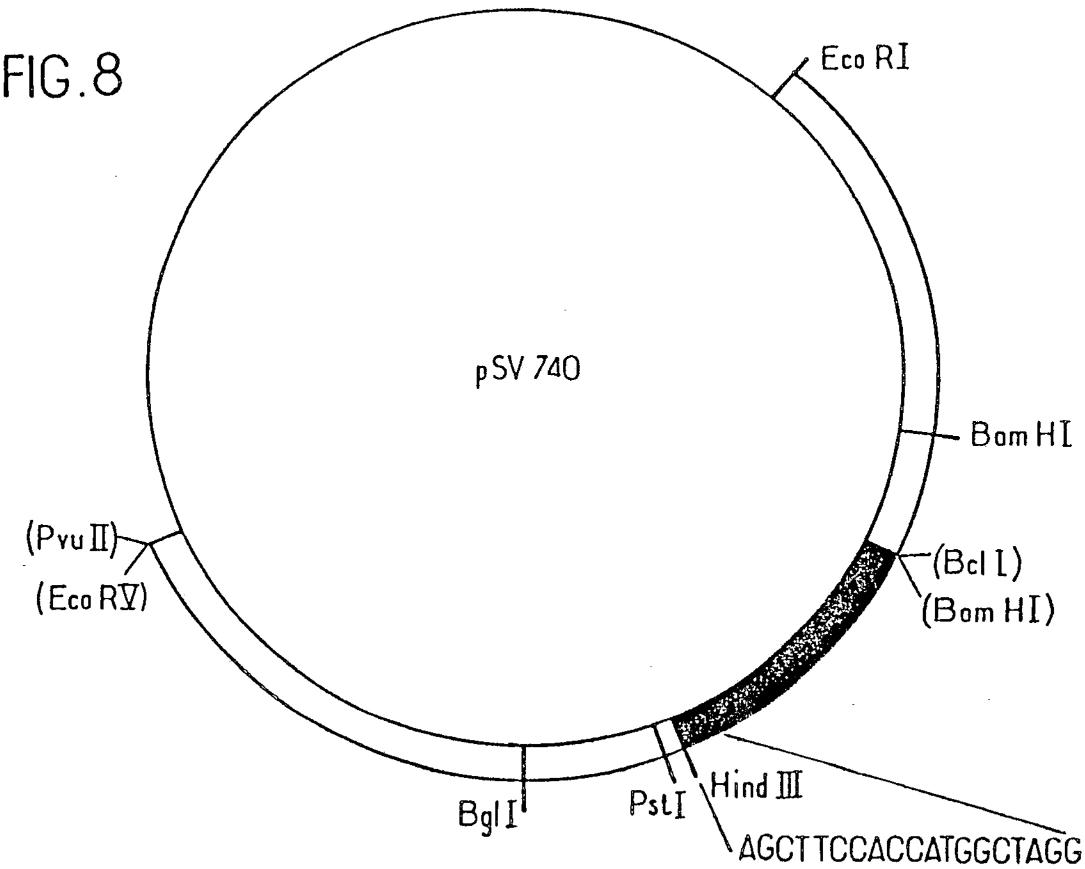
Eco RI
pSV 740
Bam HI
(Bcl I)
(Bam HI)
(Pvu II)
(Eco RV)
Bgl I
Pst I
Hind III
AGCTTCCACCATGGCTAGG

FIG. 10

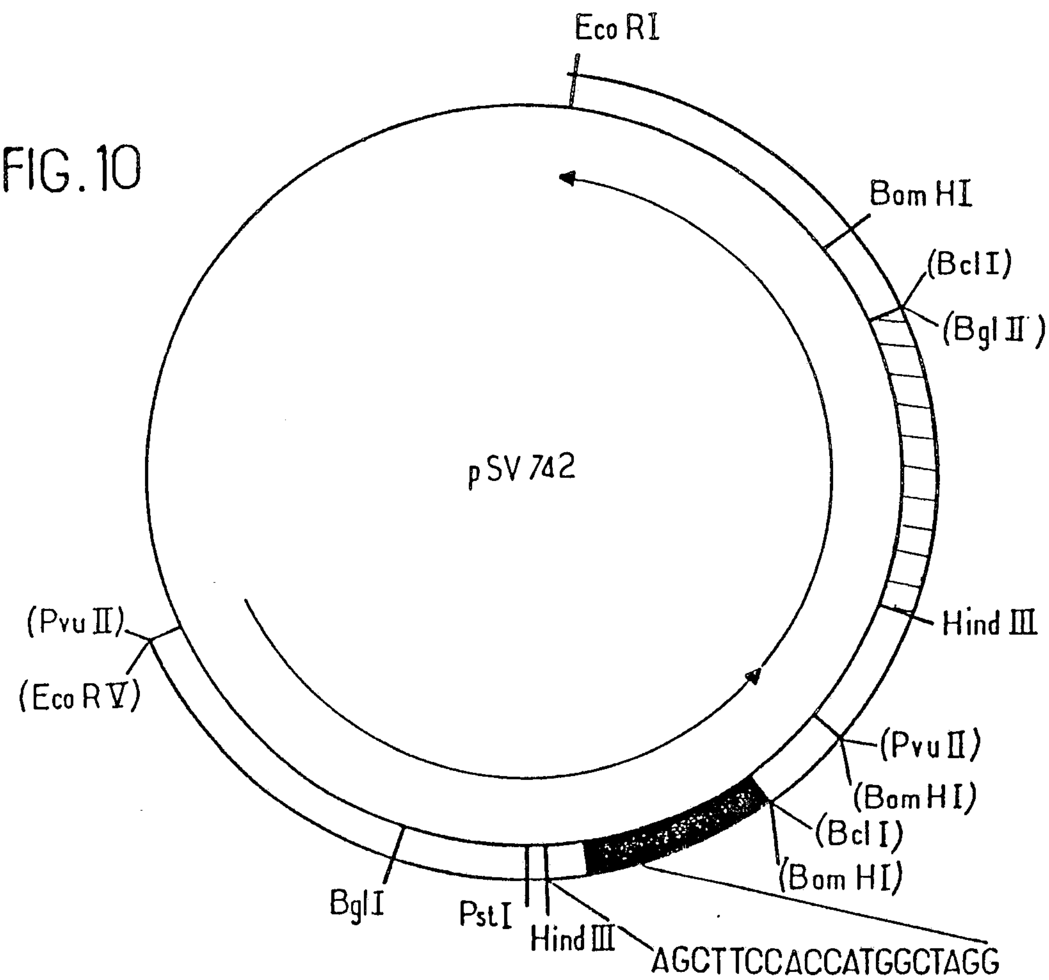
Eco RI
Bam HI
(Bcl I)
(Bgl II)
pSV 742
Hind III
(Pvu II)
(Eco RV)
(Pvu II)
(Bam HI)
(Bcl I)
(Bam HI)
Bgl I
Pst I
Hind III
AGCTTCCACCATGGCTAGG

FIG. 11

| | | | | | | | | | |
|---|---|---|---|---|---|---|---|---|---|
| | Met | Ala | Thr | Gly | Ser | Arg | Thr | Ser | Leu |
| 5' AGCTTACC | ATG | GCT | ACA | GGC | TCC | CGG | ACG | TCC | CTG |
| | Leu | Leu | Ala | Phe | Gly | Leu | Leu | Cys | Leu |
| | CTC | CTG | GCT | TTT | GGC | CTG | CTC | TGC | CTG |
| | Pro | Trp | Leu | Gln | Glu | Gly | Ser | Ala | Ala 3' |
| | CCC | TGG | CTT | CAA | GAG | GGC | AGT | GCT | GCA |

FIG.12

```
                 -20
                 Met Ala Arg Met Gln Leu Leu Ser Cys Ile Ala
5'▲ AGCTTCCACC  ATG GCT AGG ATG CAA CTC CTG TCT TGC ATT GCA
Hind III
                                     -1   1
Leu Ser Leu Ala Leu Val Thr Asn Ser Ala Pro Thr Ser Ser Ser
CTA AGT CTT GCA CTT GTC ACA AAC AGT GCA▲CCT ACT TCA AGT TCT
                                       HgiAI

Thr Lys Lys Thr Gln Leu Gln Leu Glu His Leu Leu Leu Asp Leu
ACA AAG AAA ACA CAG CTA CAA CTG GAG CAT TTA CTT CTG GAT TTA

Gln Met Ile Leu Asn Gly Ile Asn Asn Tyr Lys Asn Pro Lys Leu
CAG ATG ATT TTG AAT GGA ATT AAT AAT TAC AAG AAT CCC AAA CTC

Thr Arg Met Leu Thr Phe Lys Phe Tyr Met Pro Lys Lys Ala Thr
ACC AGG ATG CTC ACA TTT AAG TTT TAC ATG CCC AAG AAG GCC ACA

Glu Leu Lys His Leu Gln Cys Leu Glu Glu Glu Leu Lys Pro Leu
GAA CTG AAA CAT CTT CAG TGT▲CTA GAA GAA GAA CTC AAA CCT CTG
                           XbaI

Glu Glu Val Leu Asn Leu Ala Gln Ser Lys Asn Phe His Leu Arg
GAG GAA GTG CTA AAT TTA GCT CAA AGC AAA AAC TTT CAC TTA AGA

Pro Arg Asp Leu Ile Ser Asn Ile Asn Val Ile Val Leu Glu Leu
CCC AGG GAC TTA ATC AGC AAT ATC AAC GTA ATA GTT CTG GAA CTA

Lys Gly Ser Glu Thr Thr Phe Met Cys Glu Tyr Ala Asp Glu Thr
AAG GGA TCT GAA ACA ACA TTC ATG TGT GAA TAT GCT GAT GAG ACA

Ala Thr Ile Val Glu Phe Leu Asn Arg Trp Ile Thr Phe Cys Gln
GCA ACC ATT GTA GAA TTT CTG AAC AGA TGG ATT ACC TTT TGT CAA

                        133
Ser Ile Ile Ser Thr Leu Thr
AGC ATC ATC TCA ACA CTG ACT TGA TAATTAAGTGCTTCCCACTTAAAACATATCAG 3'
```

FIG. 13

```
                                                      -26
                                                      Met Ala
                                         5'▲AGCTTACC ATG GCT
                                          Hind III

Thr Gly Ser Arg Thr Ser Leu Leu Leu Ala Phe Gly Leu Leu Cys
ACA GGC TCC CGG ACG TCC CTG CTC CTG GCT TTT GGC CTG CTC TGC

                                -1  1
Leu Pro Trp Leu Gln Glu Gly Ser Ala Ala Pro Thr Ser Ser Ser
CTG CCC TGG CTT CAA GAG GGC AGT GCT GCA CCT ACT TCA AGT TCT

Thr Lys Lys Thr Gln Leu Gln Leu Glu His Leu Leu Leu Asp Leu
ACA AAG AAA ACA CAG CTA CAA CTG GAG CAT TTA CTT CTG GAT TTA

Gln Met Ile Leu Asn Gly Ile Asn Asn Tyr Lys Asn Pro Lys Leu
CAG ATG ATT TTG AAT GGA ATT AAT AAT TAC AAG AAT CCC AAA CTC

Thr Arg Met Leu Thr Phe Lys Phe Tyr Met Pro Lys Lys Ala Thr
ACC AGG ATG CTC ACA TTT AAG TTT TAC ATG CCC AAG AAG GCC ACA

Glu Leu Lys His Leu Gln Cys Leu Glu Glu Glu Leu Lys Pro Leu
GAA CTG AAA CAT CTT CAG TGT▲CTA GAA GAA GAA CTC AAA CCT CTG
                            XbaI

Glu Glu Val Leu Asn Leu Ala Gln Ser Lys Asn Phe His Leu Arg
GAG GAA GTG CTA AAT TTA GCT CAA AGC AAA AAC TTT CAC TTA AGA

Pro Arg Asp Leu Ile Ser Asn Ile Asn Val Ile Val Leu Glu Leu
CCC AGG GAC TTA ATC AGC AAT ATC AAC GTA ATA GTT CTG GAA CTA

Lys Gly Ser Glu Thr Thr Phe Met Cys Glu Tyr Ala Asp Glu Thr
AAG GGA TCT GAA ACA ACA TTC ATG TGT GAA TAT GCT GAT GAG ACA

Ala Thr Ile Val Glu Phe Leu Asn Arg Trp Ile Thr Phe Cys Gln
GCA ACC ATT GTA GAA TTT CTG AAC AGA TGG ATT ACC TTT TGT CAA

                        133
Ser Ile Ile Ser Thr Leu Thr
AGC ATC ATC TCA ACA CTG ACT TGA TAATTAAGTGCTTCCCACTTAAAACATATCAG 3'
```

Office européen des brevets

# RAPPORT DE RECHERCHE EUROPEENNE

Numero de la demande

EP 88 40 2198

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | EP-A-0 172 619 (TAKEDA CHEMICAL INDUSTRIES)<br>* Revendications; page 37, ligne 1 - page 38, ligne 14 * | 1-8,10-12,14,15 | C 12 N 5/00<br>C 12 N 15/00<br>C 12 P 21/02 |
| Y | THE JOURNAL OF BIOCHEMISTRY, vol. 102, no. 1, juillet 1987, pages 123-131, Tokyo, JP; K. ONOMICHI et al.: "Expression of amplified cDNA and genomic sequences encoding human interleukin 2 in chinese hamster ovary cells"<br>* En entier * | 1-8,10-12,14,15 | |
| Y | PROC. NATL. ACAD. SCI. USA, vol. 84, no. 9, mai 1987, pages 2638-2642, Washington, DC, US; M.L. BAYNE et al.: "Expression of a synthetic gene encoding human insulin-like growth factor I in cultured mouse fibroblasts"<br>* En entier * | 1-8,10-12,14,15 | DOMAINES TECHNIQUES RECHERCHES (Int. Cl.4) |
| P,Y | GB-A-2 190 382 (HOFFMAN-LA ROCHE & CO. AG)<br>* Revendications * | 1-8,10-12,14,15 | C 12 N |

Le présent rapport a été établi pour toutes les revendications

| Lieu de la recherche | Date d'achèvement de la recherche | Examinateur |
|---|---|---|
| LA HAYE | 07-11-1988 | HUBER-MACK A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)